(19) Europäisches Patentamt
European Patent Office
Office européen des brevets

(11) Veröffentlichungsnummer: **0 026 344**
**A1**

(12) # EUROPÄISCHE PATENTANMELDUNG

(21) Anmeldenummer: 80105220.0

(22) Anmeldetag: 02.09.80

(51) Int. Cl.³: **C 07 C 49/417**
C 07 C 49/613, C 07 C 35/21
C 07 C 45/61, C 07 C 29/136
A 61 K 7/46, C 11 B 9/00

(30) Priorität: 04.09.79 DE 2935683

(43) Veröffentlichungstag der Anmeldung:
08.04.81 Patentblatt 81/14

(84) Benannte Vertragsstaaten:
AT CH DE FR GB LI NL

(71) Anmelder: Dragoco Gerberding & Co. GmbH
Dragocostrasse 1
D-3450 Holzminden(DE)

(72) Erfinder: Brunke, Ernst-Joachim, Dr.
Holbeinstrasse 6
D-3450 Holzminden(DE)

(72) Erfinder: Klein, Erich, Dr.
Wiesenweg 50
D-3450 Holzminden(DE)

(74) Vertreter: Deufel, Paul, Dr. et al,
Patentanwälte MÜLLER-BORE-DEUFEL-SCHÖN-HERTEL Siebertstrasse 4
D-8000 München 86(DE)

(54) **2,2,3-Trimethylcyclopent-3-en-ylmethyl-substituierte alicyclische Ketone und Alkohole, Herstellungsverfahren und deren Verwendung als Riechstoffe.**

(57) Die Erfindung betrifft Verbindungen der allgemeinen Formel I

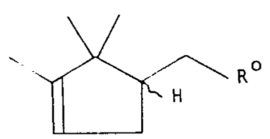

worin R° die Gruppen

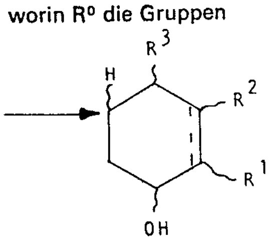
oder
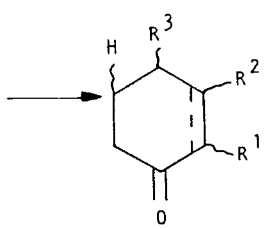

worin R¹, R² und R³ Wasserstoff, Methyl- oder Ethylgruppen darstellen und die gestrichelten Linien eine fakultative Doppelbindung und die geschlängelten Linien stereoisomere Formen bedeuten, oder

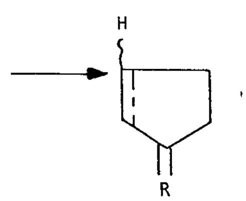,

worin R einen Carbonylsauerstoff oder eine Hydroxylgruppe und geminalen Wasserstoff bedeuten, die gestrichelte Linie eine fakultative Doppelbindung und die geschlängelten Linien stereoisomere Formen bedeuten sowie deren Herstellung in an sich bekannter Weise ausgehend von Campholenaldehyd und ihre Verwendung als Riechstoffe oder Bestandteil von Riechstoffkompositionen.

EP 0 026 344 A1

Croydon Printing Company Ltd.

# 2,2,3-Trimethylcyclopent-3-en-ylmethyl-substituierte alicyclische Ketone und Alkohole, Herstellungsverfahren und deren Verwendung als Riechstoffe

Das aus dem weißen Sandelholz-Baum (Santalum album L.) gewonnene ätherische Öl ist eine der begehrtesten und in großen Mengen eingesetzte Parfümerie-Zutaten. Als geruchsprägende Inhaltsstoffe sind α-Santalol und β-Santalol (Schema 1) erkannt worden, die zu 80-90 % im Sandelholzöl enthalten sind (Übersicht in: Gildemeister, E., und Hoffmann, Fr., "Die ätherischen Öle", Akademie Verlag, Berlin 1962, Vol.IIIb, S.329-95; Vol.IV, S.556-74).

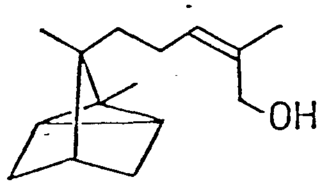

α -Santalol

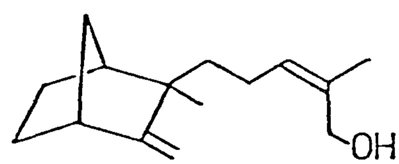

β -Santalol

- Schema 1 -

Für diese Substanzen sind verschiedene Synthesen beschrieben worden (Übersicht in: Ap Simon, Total Synthesis of Natural Products, Vol. II, S.481-91, J. Wiley, New York 1973), die aber nicht industriell ausgewertet werden können.

Es ist anzustreben, Riechstoffe mit den olfaktorischen Eigenschaften der Santalole zu synthetisieren, ohne jedoch die chemisch wenig stabile Partialstruktur der Santalol-Seitenketten einzubeziehen. Ein derartiges, zufällig gefundenes Produkt, das auf dem Riechstoff-Markt weit verbreitet ist, ist ein Gemisch von Terpenylcyclohexanolen, das durch Umsetzung von Camphen mit Phenol und anschließende Hydrierung gewonnen wird. Eingehende Untersuchungen haben gezeigt, daß nur sehr geringe Mengen an Sandelholzriechstoffen enthalten sind, die aber sehr intensiv wirken (Demole, E., Helv. Chim. Acta 47, 319, 1766 [1964]).

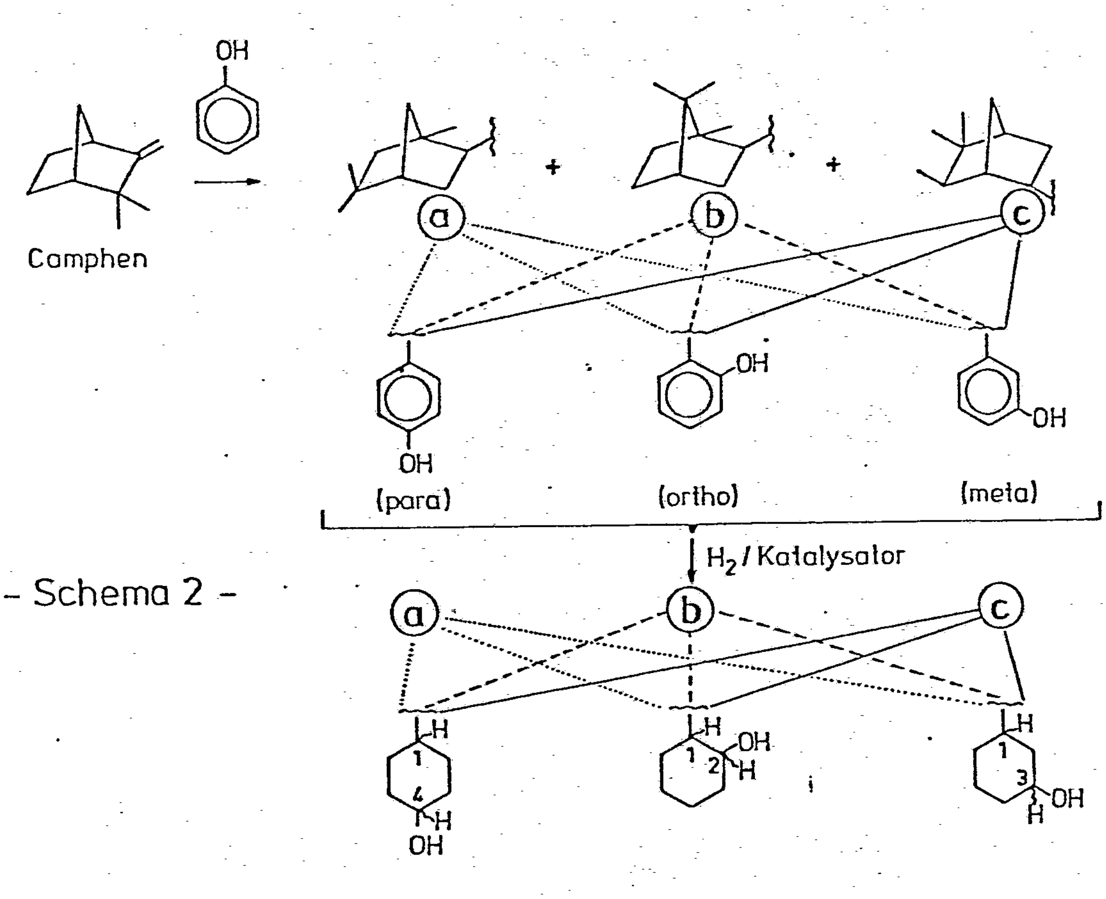


Camphen

- Schema 2 -

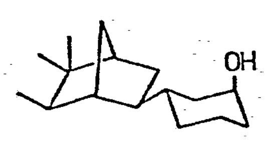

I

threo/axial

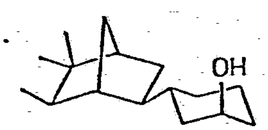

II

erytho/axial

Bei der säurekatalysierten Umsetzung von Camphen und Phenol erfolgt am Phenol-System eine Friedel-Crafts-Substitution unter Ausbildung der ortho-, para- und - in sehr geringem Maße (< 1 %) - metasubstituierten Produkte. Außerdem erfährt das Camphen-Gerüst Umlagerungen zu den Systemen a, b, c; von den durch Kombination der Partialstrukturen möglichen neun Partialstrukturen sind sechs im Reaktionsgemisch nachgewiesen worden. Bei der Hydrierung dieser Mischung entstehen die entsprechenden Cyclohexanole als Stereoisomeren-Gemische. Nur die axial 1,3-disubstituierten Isomeren I und II mit der angegebenen Stereochemie, die lediglich in Spuren enthalten sind, besitzen die gewünschte Sandelholznote. Alle übrigen Stereoisomeren und Konstitutionsisomeren sind entweder geruchlos oder nur sehr wenig wirksam (Demole, E., Helv. Chim. Acta 52, 2065 [1969]).

Die Erhöhung des Anteils von I/II soll durch Einsatz substituierter Phenole (Aulchenko, I.S., und Kheifits, L.A., Amer. Perf. and Cosm. 85, 37 [1970]), durch Verwendung bestimmter Katalysatoren oder Hydrierungsbedingungen bewirkt werden (Stern, A., und Dunkel, M., U.S. Patent 3 920 758 [Jan. 10, 1972]. Hall, J. B., und Wiegers, W.J. U.S. Patent 4 104 203 [Aug. 1, 1978]. Mulder, A.J., Dt. Offenleg. 2 732 830 [26.1.1978]). Eine gezielte Synthese zum Aufbau derartiger cyclischer Systeme (Demole, E., Brit. Pat. 1 084 235 [20.9.1967]) hat keine industrielle Bedeutung erlangt.

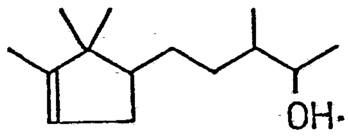

+

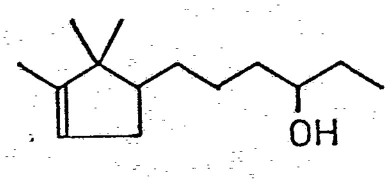

IIIa

IIIb

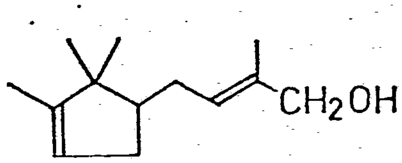

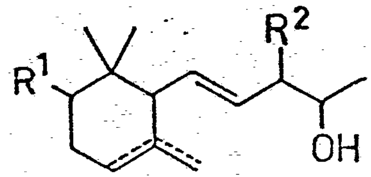

($R^1$=H,Me; $R^2$=$C_{1-6}$-Alkyl)

IV

V

- Schema 3 -

Als monocyclische Riechstoffe vom Sandelholz-Typ mit einem 2,2,3-Trimethylcyclopent-3-en-1-yl-Rest sind IIIa, b beschrieben worden, die als Gemisch entstehen und Moschus-artige Nebennoten aufweisen (Naipawer, R.E., und Easter, W.M., U.S. Patent 4 052 341 [Oct. 4, 1977]) und IV (Mühlstedt, M., Dollase, W., Herrmann, M., und Fenstel, G., Dt. Offenleg. 1 922 391 [2. Mai 1969]). Die Jonon- bzw. Iron-Derivate IV (Naegeli, P., U.S. Patent 4 046 716 [Sept. 6, 1977]) weisen blumige und sandelholzartige Noten auf.

Überraschenderweise wurde von uns gefunden, daß auch cyclische Reste, die Hydroxylgruppen enthalten, in Kombination mit dem 2,2,3-Trimethylcyclopent-3-en-1-yl-Rest (Schemata 5-10) sehr intensive und langanhaftende Sandelholz-Duftnoten aufweisen. Dies ist umso erstaunlicher,

da doch bei den Terpenylcyclohexanolen (Schema 2) die Art des bicyclischen Terpenylrestes von ausschlaggebender Bedeutung war und nur der Isocamphylrest zu Sandelholz-Noten geführt hat. Weiterhin war überraschend, daß die Einführung von Niederalkylsubstituenten an C-4', C-5' und C-6' im Cyclohexanol-Rest (Schemata 6-8) die Geruchseigenschaften vom Sandelholz-Typ und die Haftfestigkeit in sehr gewünschter Weise verbessert. Neuartig und überraschend war auch, daß die Substitution des 2,2,3-Trimethylcyclopent-3-en-1-yl-Systems mit dem 1'-Hydroxy-cyclopentyl-3'-methyl-Rest (Schema 10) zu einem Produkt mit Sandelholz-Duft führt.

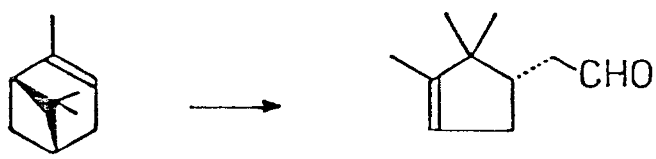

(+)-α-Pinen (+)-1 — (−)-Campholenaldehyd (−)-2

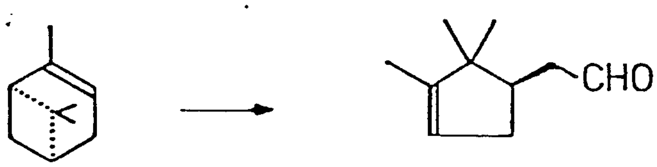

(−)-α-Pinen (−)-1 — (+)-Campholenaldehyd (+)-2

-Schema 4 -

Alle im Folgenden beschriebenen Produkte sind ausgehend von Campholenaldehyd synthetisiert worden, der in seinen enantiomeren Formen (+)-2, bzw. (-)-2 aus optisch aktivem α-Pinen (+1 bzw. -1) durch Umlagerung des Epoxids zugänglich ist (Lewis, J.B., und Hendrick, G.W., J. Org. Chem. 30, 4271 [1965]). Die enantiomeren α-Pinene 2a/2b sind als Bestandteile verschiedener Terpentinöle in großen Mengen verfügbar.

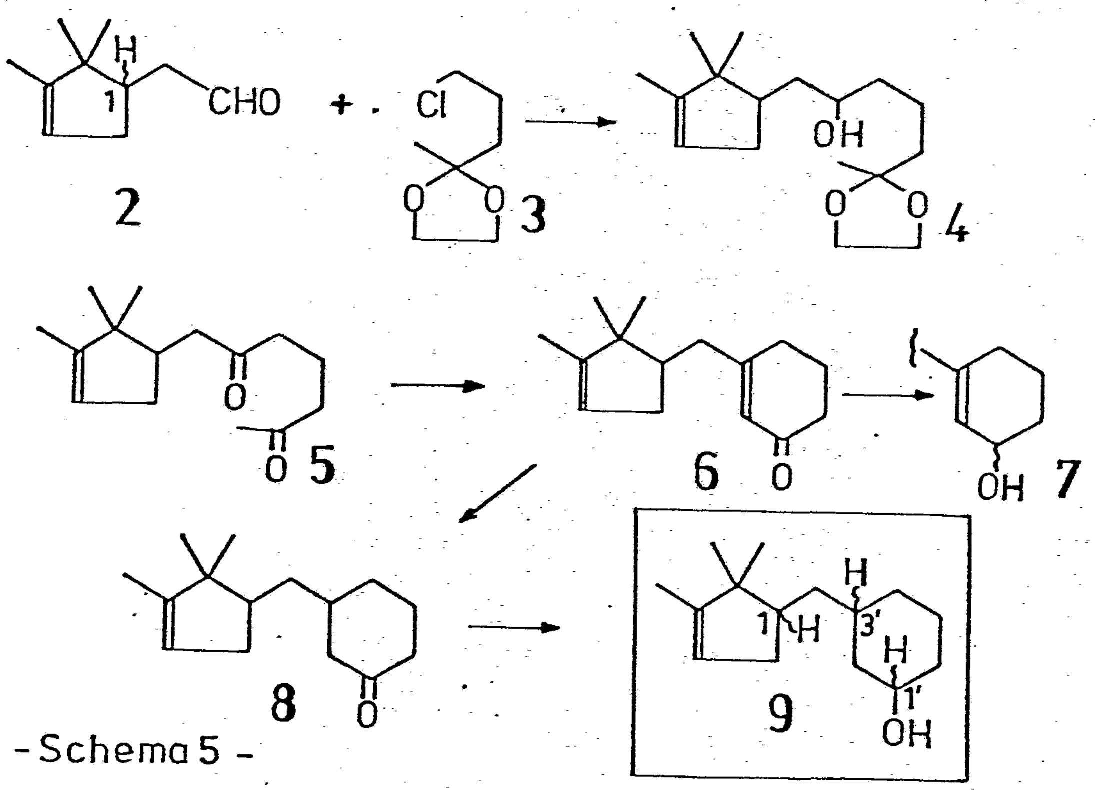

2 3 4 5 6 7 8 9

-Schema 5-

Für den Aufbau eines cyclischen Substituenten (Schema 5) wurde zunächst Campholenaldehyd (2) in bekannter Weise (Saucy, G., et al., Helv. Chim. Acta 55, 249 [1972]) mit 3, dem Ethylenketal von 1-Chlor-pentanon-4, in einer Grignard-Reaktion zu 4 umgesetzt. Die Oxidation von 4, beispielsweise mit Chromsäure unter üblichen Bedingungen auszuführen, verlief unter Acetalspaltung und ergab das

Diketon 5. Aus 5 entstand bei der Aldolkondensation unter Verwendung von starken Basen (z.B. Hydroxiden der Alkali- und Erdalkalimetalle) und polaren aprotischen Lösungsmitteln (z.B. niedrigen Alkanolen) das Cyclohexenon 6. Der durch Reduktion von 6 erhaltene Allylalkohol 7 besitzt eine kräftige Sandelholznote. Durch Hydrierung von 6 unter üblichen Bedingungen (z.B. mit Raney-Nickel oder Edelmetall-Katalysatoren) in neutralem Medium (z.B. niedrige Alkohole und Ether) kann das gesättigte Keton 8 und hieraus, z.B. durch Verlängerung der Hydrierungszeit oder Verwendung von gemischten Hydriden ($NaBH_4$, $LiAlH_4$) unter üblichen Reaktionsbedingungen das Cyclohexanol-Derivat 9 erhalten werden, wobei stereoisomere Formen an C-1, C-1' und C-3' vorliegen. 9 besitzt einen starken, leicht animalischen Sandelholz-Duft.

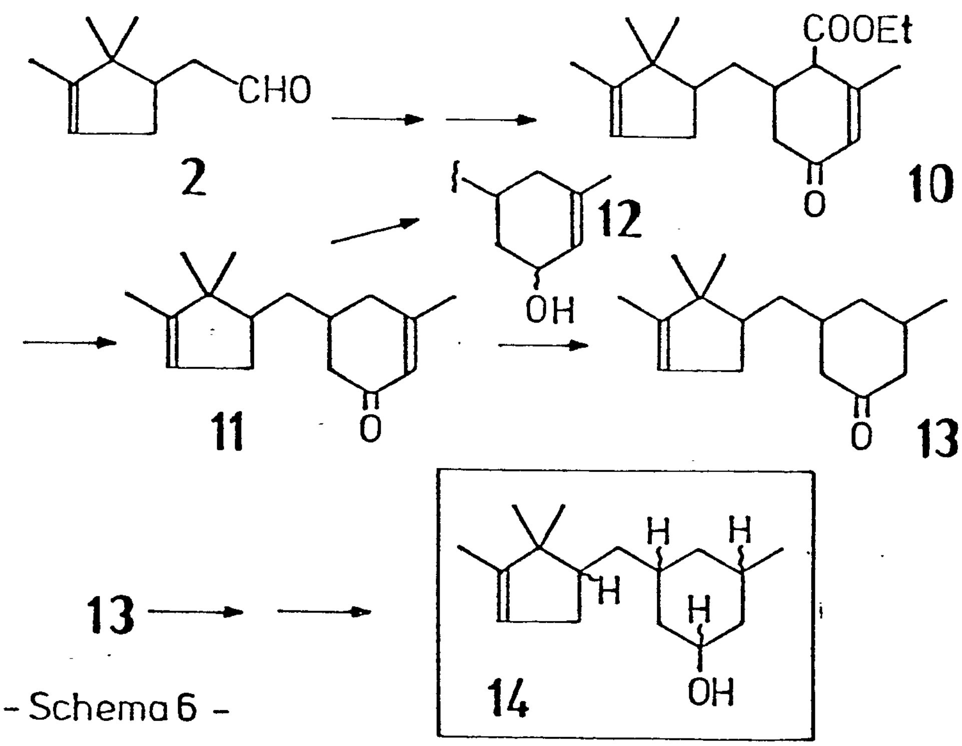

2 10 12 11 13 13 14

- Schema 6 -

Für die Einführung der Methylgruppe an C-3' des 1'-Hydroxy-

Cyclohexyl-Restes wurde die folgende Synthese des Ringsystems ausgeführt (Schema 6). Campholenaldehyd (2) wurde mit Acetessigester nach bekannter Vorschrift kondensiert (Horning, E.C., Denekas, M. O., und Field, R.E., J. Org. Chem. 9, 547 [1944]) und ergab nach partieller Verseifung und Decarboxylierung den Ketoester 10. Vollständige Verseifung und Decarboxylierung erfolgten mit ethanolischen Lösungen von Alkalihydroxiden und führte zum Cyclohexenon 11 mit angenehm holziger Duftnote. Reduktion von 11, unter üblichen Bedingungen, z.B. mit Lithiumaluminiumhydrid in Diethylether, ergab den Allylalkohol 12 mit weicher Sandelholznote. Durch katalytische Hydrierung von 11 unter Verwendung üblicher Reaktionsbedingungen und Katalysatoren (z.B. Raney-Nickel oder Palladium-Kohle) wurde durch Aufnahme äquivalenter Wasserstoffmengen das Cyclohexanon 13, bzw. durch Aufnahme von 2 Äquivalenten Wasserstoff das Cyclohexanol 14 als Stereoisomerengemisch erhalten; 13 besitzt holzig-cedrige Geruchsnoten und 14 einen intensiven und typischen Sandelholz-Duft.

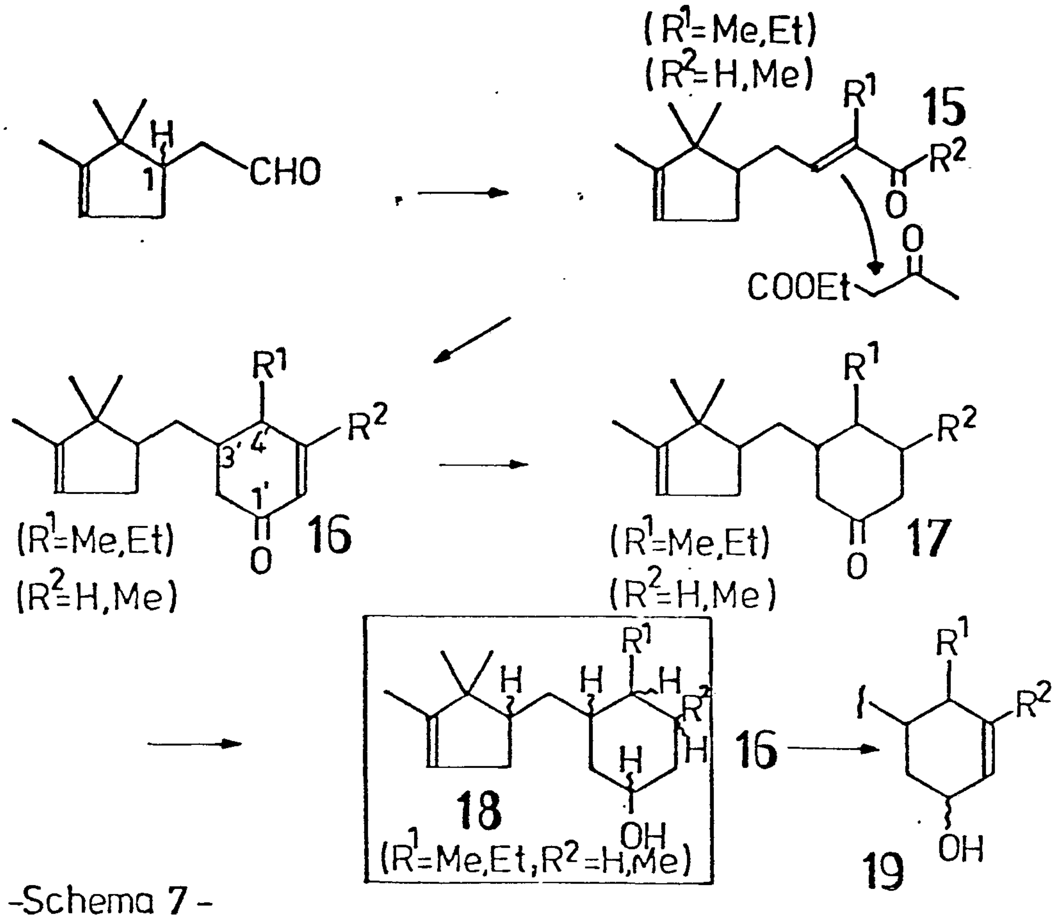

15 16 17 18 19

-Schema 7-

Die an C-4' substituierten Cyclohexanol-Derivate 18 wurden ebenfalls ausgehend von Campholenaldehyd dargestellt (Schema 7). Aldolkondensation von 2 mit Propion- oder Butyraldehyd ergab die bekannten Aldehyde 15 ($R^1$ = Me, Et, $R^2$ = H) (Dt. Offenleg. 19 22 391 [2.5.1969]), bzw. die Aldolkondensation mit Methylethylketon oder Diethylketon die Ketone 15 ($R^1$ = Me, Et; $R^2$ = Me) [Naipawer, R.E., und Easter, W.M., U.S. Patent 4 052 341; Oct. 4, 1977], die in einer Michael-Addition unter Phasentransfer-Katalyse nach an und für sich bekannter Vorschrift (Kryshtal, G.V., Kulvanev, V. V., Kucherov, V.F. und Yanovskaga, L.A., Synthesis 1979, 107) die C-4'-substituierten Cyclohexenon-Derivate 16 ergaben. Aus

16 wurden durch übliche Hydrierung die Ketone 17 (1 Äquivalent $H_2$) oder die Alkohole 18 (2 Äquivalent $H_2$) erhalten. Die Cyclohexenole 18 weisen kräftige Sandelholz-Noten auf.

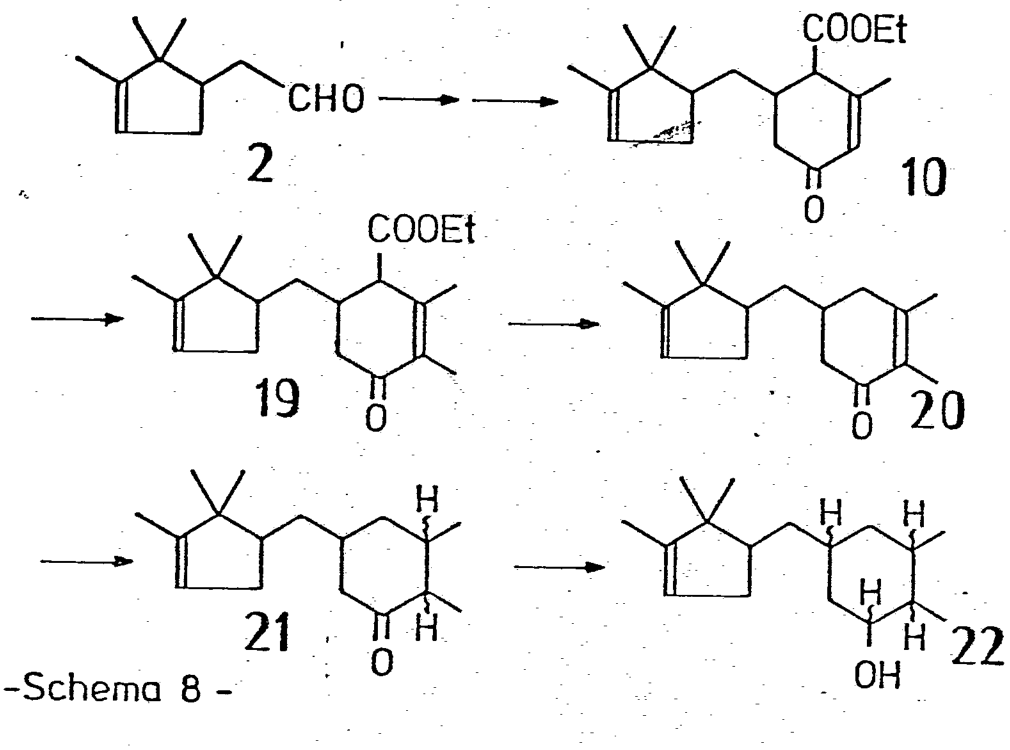

2 10 19 20 21 22

-Schema 8 -

Um den olfaktorischen Effekt einer weiteren Substitution im Hydroxycyclohexyl-Rest zu überprüfen, wurde der aus Campholenaldehyd (2) nach obengenanntem Verfahren erhaltene Keto-Ester 10 in Analogie zu der für Hagemann-Ester gegebenen Vorschrift (Smith, L.I., und Rouault, G.F., J. Amer. Chem. Soc. 65, 631 [1943]) mit Natriumethylat/Methyljodid zu 19 methyliert (Schema 8). Verseifung und Decarboxylierung nach bekannten Vorschriften ergaben das Cyclohexenon 20. Katalytische Hydrierung unter üblichen Bedingungen ergab bei Aufnahme von 1 Äquivalent Wasserstoff das Keton 21 und bei Aufnahme von 2 Äquivalent Wasserstoff das Cyclohexanol 22. 20, 21 weisen holzige Geruchsnoten und 22 solche vom Sandelholz-Typ auf, die in ihrer Geruchsstärke dem Cyclohexanol-Derivat 14 entsprechen.

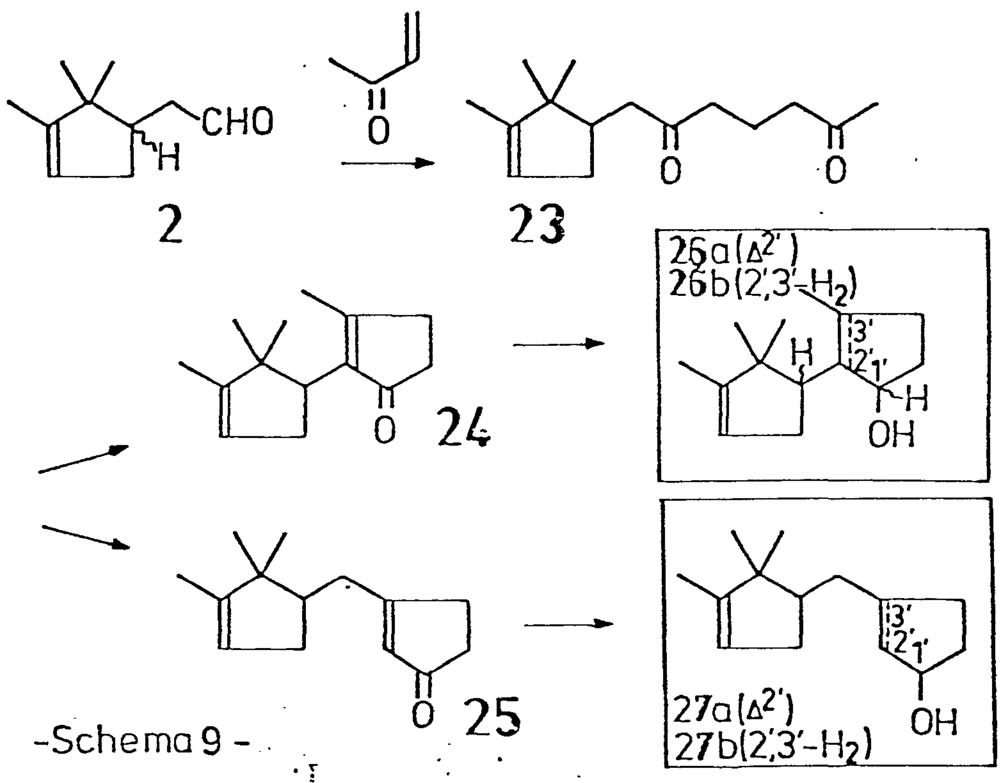

2

23

24

25

26a (Δ2')
26b (2',3'-H2)

27a (Δ2')
27b (2',3'-H2)

-Schema 9 -

Durch Umsetzung von Campholenaldehyd (2) mit Methylvinylketon (Schema 10) nach bekannter Methode (Stetter, H., und Kuhlmann, H., Synthesis 1975, 379) wurde das γ-Diketon 23 und durch dessen intramolekulare Aldolkondensation das Cyclopentenon 24 sowie das Alternativprodukt 25 erhalten, die durch fraktionierte Destillation getrennt wurden. 24 besitzt süß-krautige, an Tabak und Jasmon erinnernde Duftnoten, während 25 einen holzigen leicht urinösen Geruch aufweist. Bei der Reduktion bzw. Hydrierung unter bekannten Reaktionsbedingungen entstanden aus 24 die ungesättigten bzw. gesättigen Alkohole 26a bzw. 26b mit interessanten blumigen Geruchsnuten und aus 25 die ungesättigten bzw. gesättigten Alkohole 27a,b mit weichen, aber typischen Sandelholz-Noten.

Die beschriebenen Reaktionswege, die zu den Verbindungen der allgemeinen Formel A, B und C führen, sind Kombinationen von bekannten und teilweise einfachen Reaktionsschritten. Durch die aufgeführten Herstellungsbeispiele, die übliche Verfahrensweisen enthalten, sollen ähnliche Reaktionsmedien oder Reaktionstemperaturen oder Katalysatoren nicht ausgeschlossen werden.

Die Verbindungen der allgemeinen Formeln A, B und C deren Darstellung in den Reaktionsschemata 5 - 9 beschrieben ist, zeichnen sich durch besondere Geruchseigenschaften aus, insbesondere Verbindungen der Formel A und C durch langanhaftende, mildholzige, etwas animalische Geruchsnoten, die dem Hauptgeruchskomplex des ostindischen Sandelholzöls weitgehend entsprechen, jedoch in der chemischen Stabilität dieses ätherischen Öls bei weitem übertreffen.

Die Verbindungen der allgemeinen Formel B besitzen angenehm holzige Noten, die vor allem an Cedernholzöl, aber auch an bestimmte Noten der Ambra erinnern.

Die Verbindungen der allgemeinen Formeln A, B und C sind aufgrund ihrer interessanten Geruchsnoten und ihrer chemischen Stabilität für die Verwendung als Riechstoffe und somit als Bestandteile von Riechstoffkompositionen wie Parfums und Parfumbasen, bzw. zur Parfumierung kosmetischer und technischer Produkte aller Art geeignet.

Die folgenden Beispiele beschreiben die Herstellung der Verbindungen der allgemeinen Formeln A, B und C.

Für alle neuen Verbindungen (4-27a, b) liegen zutreffende IR-$^1$H-NMR- und Massen-Spektren vor, von denen im folgenden nur signifikante Daten aufgeführt werden.

Herstellungsbeispiel 1 (zu Schema 5)

3-(2',2',3'-Trimethyl-cyclopent-3'-en-1'-yl)-methyl-cyclohex-2-en-1-on (6) : Zu einer aus 6 g (0,25 mol) Magnesium (als Späne) und 41 g (0,25 mol) 1-Chlor-4-ethylendioxy-pentan in 250 ml absol. Tetrahydrofuran (THF) bereiteten Grignard-Lsg. wurden 38 g (0,25 mol) Campholenaldehyd, in 100 ml absol. THF gelöst, hinzugetropft. Nach 1 h Rühren bei Siedetemperatur wurde hydrolysiert und nach Sättigung der wässrigen Phase mit $NH_4Cl$ aufgearbeitet. Das Rohprodukt (71 g braunes Öl) wurde in 100 ml Ether gelöst und mit einer Lösung von 32 g Natriumdichromat in 160 ml Wasser und 24 ml konz. Schwefelsäure unter kräftigem Rühren vermischt. Das nach Aufarbeiten der organischen Phase vorliegende Rohprodukt 5 (62 g braunes Öl, IR: 1710 $cm^{-1}$) wurde in 500 ml 1 % ethanolischer Natronlauge 1 h zum Sieden erhitzt. Nach Abdestillieren des Ethanols wurde aufgearbeitet und durch Säulenchromatographie an 800 g Kieselgel (Ø = 4 cm, Elution mit Petrolether/Methylenchlorid 2:1) gereinigt. Aus der Hauptelution wurden 14 g gaschromatographisch einheitliches 6 erhalten, aus weiteren Elutionen 40 g 6 mit ca. 20 % Nebenprodukten.

NMR: 0,93 + 1,12 (2s, 2', 2'-$CH_3$), 1,62 ("s", 3'-$CH_3$), 5,16 (m, 4'-H), 5,72 ppm (m, 2-H), - IR: 1680, 1630 $cm^{-1}$.

MS: m/e (%) = 218 (22, $M^+$), 203(16), 200(3), 185(15), 175(7), 162(11), 149(10), 122(23), 108(100).

$C_{15}H_{22}O$ (218.33).

Herstellungsbeispiel 2 ( zu Schema 5)

3-(2',2',3'-Trimethyl-cyclopent-5'-en-1'-yl)-methyl-cyclohex-2-en-1-ol (7): Einer Aufschlämmung von 0,44 g (0,012 mol) Lithiumaluminiumhydrid in 60 ml absol. Ether wurde eine Lösung von 6 g 6 in 40 ml absol. Ether unter Rühren zugetropft. Nach 1 h Rühren bei Siedetemperaturen wurde vorsichtig Wasser zugegeben. Aufarbeitung erbrachte 6 g 7 als farbloses Öl. - NMR: δ = 0,95 + 1,14, 2s (2',2'-$CH_3$), 1,60 "s" (3'-$CH_3$), 3,9 - 4,3, m (1-H), 5,20, m (4'-H), 5,50 ppm, m (2-H). IR: 3350 $cm^{-1}$ (s). MS: m/e (%) = 220 (6, $M^+$), 202 (28), 187 (9), 159(8), 145(5), 131(9), 121(12), 108(100). - $C_{15}H_{24}O$ (220.34).

Herstellungsbeispiel 3 (zu Schema 5)

3-(2',2',3'-Trimethyl-cyclopent-3'-en-1'-yl)-methyl-cyclohexan-1-on (8) : 10 g 6 wurden in 60 ml absol. Methanol mit 0,2 g Raney-Nickel unter Normalbedingungen hydriert, bis 1 Äquivalent Wasserstoff aufgenommen war. Nach Filtration und Einengen lagen 10 g 8 als farbloses Öl vor. - IR: 1715 $cm^{-1}$ . - $C_{15}H_{24}O$ (220.34).

Herstellungsbeispiel 4 (zu Schema 5)

3-(2',2',3'-Trimethyl-cyclopent-3'-en-1'-yl)-methylcyclohexanol-1(9): 10 g 6 wurden in 60 ml absol. Methanol mit 0,2 g Raney-Nickel unter Normalbedingungen hydriert, bis 2 Äquivalent Wasserstoff aufgenommen war. Es wurden nach Filtration und Einengen 10 g 9 als farbloses Öl erhalten. - IR: 3350 $cm^{-1}$. - $C_{15}H_{26}O$ (222.36).

Herstellungsbeispiel 5 ( zu Schema 6)

3-Methyl-5-(2',2',3'-trimethyl-cyclopent-3'-en-1-yl)-methyl-cyclohex-2-en-1-on (11) : Zu einer Mischung von 152 g (1 mol) Campholenaldehyd (2) und 328 g (2,5 mol) Acetessigester wurden bei 0°C 10 ml Piperidin, in 20 ml Ethanol, gelöst, unter Rühren zugetropft. Nach 3 Tagen Stehen bei Raumtemperatur wurde 4 h unter Rückfluß gekocht. Der nach Abdestillieren des Ethanols vorliegende Ester 10 (IR: 1730, 1670, 1630 $cm^{-1}$) wurde mit 100 g NaOH und 2 l 90 % Isopropanol versetzt und 24 h bei Siedetemperatur gerührt. Das durch Einengen gewonnene Rohprodukt wurde in Methylenchlorid aufgenommen, mit 10%iger $H_2SO_4$ und hiernach mit Wasser neutralgewaschen, aufgearbeitet und destilliert. Man erhielt 107 g (46 %) 11. Kp (1 mm) 120-123°C. - NMR: 0,75 + 0,97 (2s, 2',2'-$CH_3$), 1,60 ("2", 3'-$CH_3$), 1,92 ("s", 3-$CH_3$), 5,15 (m, 4-H), 5,70 ppm (m, 2-H). - IR: 1670, 1632 $cm^{-1}$. - $C_{16}H_{24}O$ (232.35).

Herstellungsbeispiel 6 (zu Schema 6)

3-Methyl-5-(2',2',3'-trimethyl-cyclopent-3'en-1'-yl)-methyl-cyclohex-2-en-1-ol (12): Aus 10 g 11 wurden analog dem Herstellungsbeispiel 2 9,2 g (90 %) 12 erhalten. NMR: 0,75 + 0,97 (2s, 2',2'-$CH_3$), 1,60-1,65 (br."s", 3'-$CH_3$ + 3-$CH_3$), 3,85 - 4,4 (m, 1-H), 5,15 (m, 4'-H), 5,35 ppm (m, 2-H). - IR: 3320 $cm^{-1}$ - $C_{16}H_{26}O$ (234.37).

Herstellungsbeispiel 7 (zu Schema 6)

3-Methyl-5-(2',2',3'-trimethyl-cyclopent-3'-en-1'-yl)-methyl-cyclohexan-1-on (13): Aus 10 g 11 wurden analog dem Beispiel 3 hydriert und ergaben 10 g 13 als farbloses Öl. - IR: 1710 $cm^{-1}$. - $C_{16}H_{26}O$ (234.37).

Herstellungsbeispiel 8 (zu Schema 6)

3-Methyl-5-(2',2',3'-trimethyl-cycopent-3'-en-1'-yl)-methyl-cyclohexan-1-ol (14): Wie in Beispiel 4 für 9 beschrieben, wurden aus 20 g 13 nach Destillation 18 g 14, Kp (1 mm) = 124 - 126°C. NMR: 0,73 + 0,99 (2s, 2',2'-$CH_3$), 1,02 (d, 3-$CH_3$), 1,60 ("s",3'-$CH_3$), 5,15 ppm (m, 4'-H). - IR: 3400 $cm^{-1}$. $C_{16}H_{28}O$ (236,38).

Herstellungsbeispiel 9 (zu Schema 7)

5-(2',2',3'-Trimethyl-cyclopent-3'en-1'-yl)-methyl-4-methyl-cyclohex-2-en-1-on (16, $R^1$ = Me, $R^2$ = H): Bei 40-45°C wurde zu einer Suspension von 138,2 g (1 mol) Kaliumcarbonat und 4,7 g (0,02 mol) Triethylbenzylammoniumchlorid (TEBAC) in 130 g (1 mol) Acetessigsäureethylester und 400 ml Benzol eine Lösung von 192 g (1 mol) 1-(3'-Formyl-but-2'-enyl)-2,2,3-trimethyl-cyclopent-3-en (15, R = Me) zugetropft. Nach 4 h Rühren bei 40-45°C wurde 30 min zum Sieden erhitzt. Filtration und Aufarbeitung des Filtrats ergaben 318 g Rohprodukt, aus dem durch fraktionierte Destillation 120,5 g (52 %) 16 als farbloses Öl gewonnen wurden (2 Stereoisomere, ca. 95:5); Kp (0,8 mm) 110°-114°C - NMR: 0,77 + 0,98 (2s, 2',2'-$CH_3$), 1,18 + 1,22 (2d, J = 6 $H_2$, 4α- bzw. 4β-$CH_3$), 1,60 ("s", 3'-$CH_3$), 5,16 (m, 4'-H), 5,6 - 6,7 ppm (m, 2-, 3-H). - IR: 1680, 1620 $cm^{-1}$. - $C_{16}H_{24}O$ (232,35).

Herstellungsbeispiel 10 (zu Schema 7)

5-(2',2',3'-Trimethyl-cyclopent-3'-en-1'-yl)-methyl-4-methyl-cyclohexanon-1 (17, $R^1$ = Me, $R^2$ = H): 11,6 g (0,05 mol) 16 (R = Me) wurden in 60 ml absol. Methanol mit 0,2 g Raney-Nickel unter Normalbedingungen

hydriert. Nach ca. 1 h war 1 Äquivalent Wasserstoff (1,1 l) aufgenommen. Filtration und Abdestillieren des Lösungsmittels ergaben 11,5 g 17 als farbloses Öl. Kp (1 mm) 116-118$^{O}$C. - NMR: = 0,77 (s), 0,98 (s), 1,02 (d), 1,60 ("s"), 5,17 ppm (m). - IR: 1715 $cm^{-1}$. - $C_{16}H_{26}O$ (234,37).

Herstellungsbeispiel 11 (zu Schema 7)

5-(2',2',3'-Trimethyl-cyclopent-3'-en-1'-yl)-4-methyl-cyclohexan-1-ol (18, $R^1$ = Me, $R^2$ = H): 11,6 g (0,05 mol) 16 (R = Me) wurden in 60 ml absol. Methanol mit 0,2 g Raney-Nickel unter 5 at. $H_2$ hydriert, bis 2 Äquivalent Wasserstoff aufgenommen waren. Nach Filtration und Einengen des Lösungsmittels verblieben 11,5 g 18 (R = Me) als farbloses Öl. Kp (1 mm) 132 - 134$^{O}$C. - NMR: 0,74(s), 0,90(d), 0,97(s), 1,59("s"), 3,2-3,9(m), 5,15 ppm(m).-IR: 3350 $cm^{-1}$. - $C_{16}H_{28}O$ (236,38).

Herstellungsbeispiel 12(zu Schema 7)

5-(2',2',3'-Trimethyl-cyclopent-3'-en-1'-yl)-methyl-4-methyl-cyclohex-2-en-1-ol (19, $R^1$ = Me, $R^2$ = H): Zu einer Suspension von 0,44 g (0,012 mol) $LiAlH_4$ in 60 ml absol. Ether wurde eine Lösung von 8 g (0,034 mol) 16 (R = Me) in 40 ml absol. Ether unter Rühren hinzugetropft. Nach 1 h Rühren bei Siedetemperatur, vorsichtiger Zugabe von Wasser und Aufarbeitung verblieben 8,3 g 19 als hellgelbes Öl. Kp (1,5 mm) 135 - 138$^{O}$C - NMR: 0,78(s), 1,00(s), 1,02(d), 1,61 ("s"), 5,14 (m), 5,2 - 5,7 ppm(m). - IR: 3340, 1650 $cm^{-1}$. - $C_{16}H_{26}O$ (234,37).

Herstellungsbeispiel 13 (zu Schema 8)

2,3-Dimethyl-5-(2',2',3'-trimethyl-cyclopent-3'-en-1'-yl)-methyl-cyclohex-2-en-1-on (20): Der nach Beispiel 4

erhaltene Ester 10 [152 g (0,5 mol)] wurde mit einer aus 11,5 g (0,5 mol) Natrium und 300 ml absol. Ethanol bereiteten Natriumethylat-Lösung versetzt und 1 h unter Schutzgas zum Sieden erhitzt. Nach Abkühlung wurden 71 g (0,5 mol) Methyljodid, gelöst in 100 ml Ethanol, zugetropft. Nach 20 h Rühren bei Siedetemperatur und Abziehen des Ethanols wurde aufgearbeitet. Der rohe Ester 19 wurde in 500 ml 5 % Ethanol. NaOH gelöst und 24 h unter Rühren zum Sieden erhitzt. Aufarbeiten und Destillation ergaben 72 g (62 %) 20 als hellgelbes Öl; Kp (1mm) 129 - 133°C. - NMR: 0,76 + 1,00 (2s, 2',2'-$CH_3$), 1,61 ("s", 3'-$CH_3$), 1,72 ("s", 2-$CH_3$), 1,91 ("s", 3-$CH_3$), 5,14 ppm (m, 4'-H). - IR: 1670, 1630 $cm^{-1}$. - $C_{17}H_{26}O$ (246,38).

Herstellungsbeispiel 14 (zu Schema 8)

2,3-Dimethyl-5-(2',2',3'-trimethyl-cyclopent-3'-en-1'-yl)-cyclohexan-1-ol (22): Durch Hydrierung bei erhöhtem Druck (20-40 atü) und Temperaturen zwischen 15-50°C entstanden in Abhängigkeit von der aufgenommenen Wasserstoffmenge zunächst 21 IR: 1710 $cm^{-1}$) und hiernach 22 (ca. 10 g). - IR: 3380 $cm^{-1}$. - $C_{17}H_{28}O$ (248,39).

Herstellungsbeispiel 15 (zu Schema 9)

3-Methyl-2-(2',2',3'-trimethyl-cyclopen-3'-en-1'-yl)-cyclopent-2-en-1-on (24) und 3-(2',2',3'-Trimethyl-cyclopent-3'-en-1'-yl)-methyl-cyclopent-2-en-1-on (25): Zu einer siedenden Lösung von 152 g (1 mol) Campholenaldehyd, 27 g (0,1 mol) 3-Benzyl-5-(2-hydroxyäthyl)-4-methyl-1,3-thiazolium-chlorid und 70,2 g (1 mol) Vinylmethylketon in 300 ml absol. Ethanol läßt man 60,6 g (0,6 mol) Triethylamin unter Rühren hinzulaufen. Nach 12 h Rühren bei Siedetemperatur wird die abgekühlte Lösung in

2 l 2%ige $H_2SO_4$ gegossen und aufgearbeitet. Das γ-Diketon 23 (193 g Rohprodukt) wurde mit 193 g KOH in 3 l Ethanol 1 h unter Stickstoff zum Sieden erhitzt. Der erhaltenen Reaktionslösung wurden 200 g Eisessig zugegeben. Nach Abdestillieren des Ethanols wurde aufgearbeitet. Das Rohprodukt (155 g braunes Öl) ergab nach Destillation 145 g Produktgemisch, das an einer Drehbandkolonne fraktioniert wurde. Man erhielt 50 g 24 und 13,2 g 25 gaschromatographisch einheitlich sowie Zwischenfraktionen mit Mischungen beider Substanzen.

24: NMR: δ = 0,75 + 0,98 (2s, 2',2'-$CH_3$), 1,62 ("2", 3'-$CH_3$), 2,08 (s, 3-$CH_3$), 5,23 ppm (m, 4'-H) - IR: 1698, 1630 $cm^{-1}$. - $C_{14}H_{20}O$ (204,30).

25: NMR: δ = 0,83 + 1,01 (2s, 2',2'-$CH_3$), 1,63 ("s", 3'-$CH_3$), 5,17 (m, 4'-H), 5,80 ppm (m,2-H). - IR: 1702, 1615 $cm^{-1}$. $C_{14}H_{20}O$ (204,30).

Herstellungsbeispiel 16 (zu Schema 9)

3-(2',2',3'-Trimethyl-cyclopent-3'-en-1'-yl)-methyl-cyclopentan-1-ol (27b): 5 g 25 in 50 ml Methanol wurden mit 0,3 g Raney-Nickel unter Normalbedingungen hydriert. Das nach Aufnahme von 1 Äquivalent $H_2$ vorliegende gesättigte Keton 25 (2-H,H) [IR: 1740 $cm^{-1}$] wurde mit 1 g $NaBH_4$ in 30 ml Ethanol reduziert. Man erhielt 5,1 g 27b als farbloses Öl. - IR: 3360 $cm^{-1}$. - NMR: 0,75 + 0,98 (2s,2',2'-$CH_3$), 1,62 ("s", 3'-$CH_3$), 3,9 - 4,3 (m, 1-H), 5,13 ppm (m, 4'-H). - $C_{14}H_{24}O$ (208,33).

## Patentansprüche

1. Verbindungen der allgemeinen Formel I

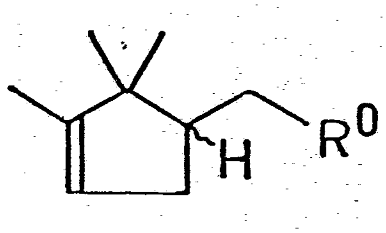

worin $R^0$ die Gruppen

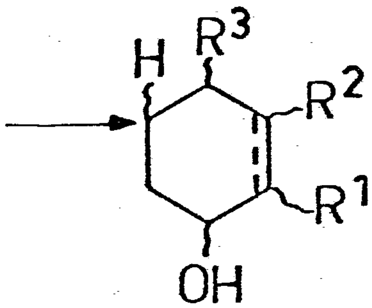

oder

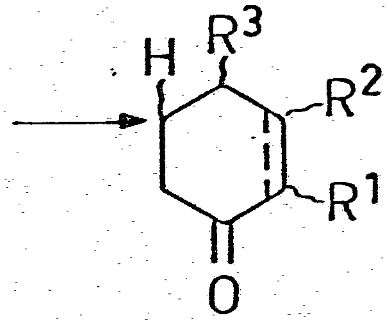

worin $R^1$, $R^2$ und $R^3$ Wasserstoff, Methyl- oder Ethylgruppen darstellen und die gestrichelten Linien eine fakultative Doppelbindung und die geschlängelten Linien stereoisomere Formen bedeuten, oder

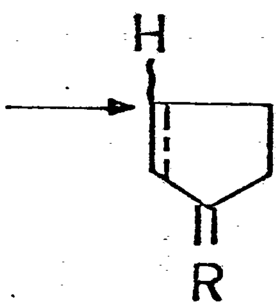

worin R einen Carbonylsauerstoff oder eine Hydroxylgruppe

und geminalen Wasserstoff bedeuten, die gestrichelte Linie eine fakultative Doppelbindung und die geschlängelten Linien stereoisomere Formen bedeuten.

2. Verbindungen nach Anspruch 1 der allgemeinen Formel A,

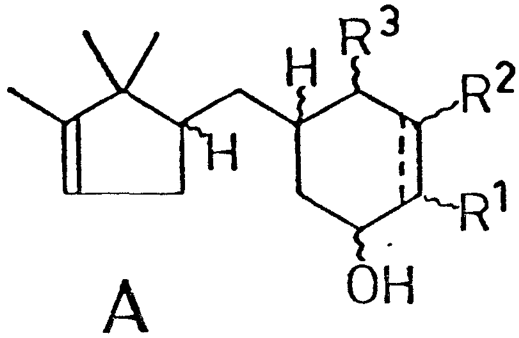

A

worin $R^1$, $R^2$ und $R^3$ Wasserstoff, Methyl- oder Ethylgruppen darstellen und die gestrichelte Linie eine fakultative Doppelbindung und die geschlängelten Linien stereoisomere Formen bedeuten.

3. Verbindungen nach Anspruch 1 der allgemeinen Formel B

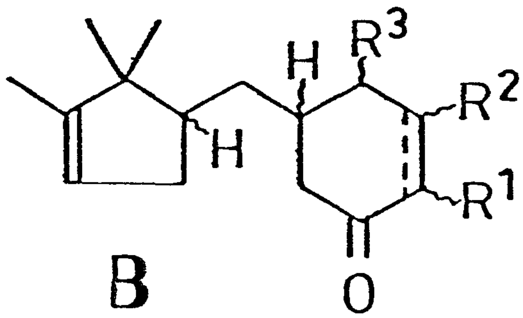

B

worin $R^1$, $R^2$ und $R^3$ Wasserstoff, Methyl- oder Ethylgruppen darstellen, die gestrichelte Linie eine fakultative Doppelbindung und die geschlängelten Linien stereoisomere Formen bedeuten.

4. Verbindungen nach Anspruch 1 der allgemeinen Formel C, worin R einen Carbonyl-Sauerstoff bzw. eine Hydroxylgruppe und geminalen Wasserstoff,

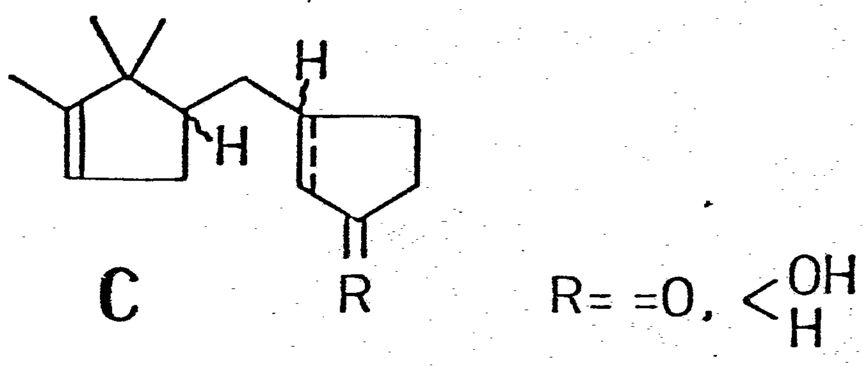

C

die gestrichelte Linie eine fakultative Doppelbindung und die geschlängelten Linien stereoisomere Formen bedeuten.

5. Verfahren zur Herstellung von Verbindungen nach Anspruch 1, dadurch gekennzeichnet, daß man Campholenaldehyd (2) mit Estern der Acetessigsäure, in an sich bekannter Weise kondensiert, den durch partielle Verseifung und Decarboxylierung erhaltenen Ketoester (10), gegebenenfalls nach Methylierung mit Na-methylat/Methyljodid; mit ethanolischer Alkalihydroxidlösung vollständig zum Cyclohexanon (11) verseift und decarboxyliert und diesen gegebenenfalls in an sich bekannter Weise, mit Lithiumaluminiumhydrid in Diethylether zum Allylalkohol (12) reduziert oder die Verbindung (11) katalytisch zum Cyclohexanon (13) oder, durch Aufnahme von zwei Äquivalenten Wasserstoff, zum Cyclohexanol (14) hydriert.

6. Verwendung der Verbindungen nach Anspruch 1 bis 4 als Riechstoffe oder Bestandteil von Riechstoff-Kompositionen.

0026344

Europäisches Patentamt

# EUROPÄISCHER RECHERCHENBERICHT

Nummer der Anmeldung

EP 80 10 5220.0

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X,P | WO - A1 - 80/00915 (FRITZSCHE DODGE & OLCOTT INC.)<br>* ganzes Dokument *<br>-- | 1-6 |
| P | GB - A- 2 024 208 (DRAGOCO GERBERDING)<br>* Anspruch 13; Seite 2, Schema 1 *<br>-- | 5,6 |
| A,P | Patents Abstracts of Japan, Band 3, Nr. 147, 5. Dezember 1979<br>Seite 102C66<br>& JP - A - 54 - 125645<br>-- | 4,6 |
| A,P | Patents Abstracts of Japan, Band 4, Nr. 69, 22. Mai 1980<br>Seite 79C11<br>& JP - A - 55 - 35041<br>-- | 4,6 |
| | JOURNAL OF THE CHEMICAL SOCIETY, CHEMICAL COMMUNICATIONS, Band 1978, Nr. 1<br>London<br>J.S. H. KUEH et al. "Photocyclisations of Dicyclopent-1-enyl Methanes to Tricyclo[6.3.0.0.$^{2,6}$]undecanes: a Synthesis of the Hirsutane Carbon Skeleton"<br>Seiten 5 und 6<br>--<br>./.. | 4 |

**KLASSIFIKATION DER ANMELDUNG (Int.Cl.3)**

C 07 C 49/417
C 07 C 49/613
C 07 C 35/21
C 07 C 45/61
C 07 C 29/136
A 61 K 7/46
C 11 B 9/00

**RECHERCHIERTE SACHGEBIETE (Int. Cl.3)**

A 61 K 7/46
C 07 C 29/136
C 07 C 35/21
C 07 C 45/61
C 07 C 49/417
C 07 C 49/613
C 11 B 9/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-12-1980 | KNAACK |

Europäisches Patentamt

**EUROPÄISCHER RECHERCHENBERICHT**

0026344

Nummer der Anmeldung

EP 80 10 5220.0



| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | JOURNAL OF ORGANIC CHEMISTRY, Band 43, Nr. 17, 1978<br>Washington D.C.<br>E. PIERS et al. "Cuprates Derived from endo-(n+3)-Bromobicyclo [n.1.0] alkanes and Related Compounds and Their Reaction with ß-Iodo Enones. Facile Homo-[1,5]-sigmatropic Hydrogen Migrations Involving endo-(n+3)-(3-Keto-1-cycloalkenyl)bicyclo [n.1.0] alkanes".<br>Seiten 3431 bis 3432<br>---- | 3,4 | |
| | | | RECHERCHIERTE SACHGEBIETE (Int. Cl.3) |